# EUROPEAN PATENT SPECIFICATION

(11) **EP 0 369 685 B1**
(45) Date of publication and mention of the grant of the patent: **19.04.1995**
(21) Application number: 89311634.3
(22) Date of filing: 10.11.1989
(51) Int. Cl.: C07C 217/48, A61K 31/135

(54) **Fluoxetine analog**
Fluoxetin-Analogon
Analogue de fluoxétine

(30) Priority: 14.11.1988 US 270177
(43) Date of publication of application: 23.05.1990
(73) Proprietor: ELI LILLY AND COMPANY, Indianapolis, Indiana 46285 (US)
(72) Inventor: Fuller, Ray Ward, Indianapolis Indiana 46227 (US); Robertson, David Wayne, Greenwood Indiana 46142 (US); Wong, David Taiwai, Indianapolis Indiana 46217 (US)
(74) Representative: Hudson, Christopher Mark

(56) References cited:
- US-A- 4 313 896
- US-A- 4 868 344
- JOURNAL OF MEDICINAL CHEMISTRY, vol. 31, no. 7, July 1988, pages 1412-1417, American Chemical Society; D.W. ROBERTSON et al.: "Absolute configurations and pharmacological activites of the optical isomers of fluoxetine, a selective serotonin-uptake inhibitor"

## Description

This invention relates to a novel analog of fluoxetine having unexpectedly advantageous properties.

During the past decade, the relationship between monoamine uptake and a variety of diseases and conditions has been appreciated and investigated. For example, the hydrochloride salt of fluoxetine (dl-N-methyl-3-[4-(trifluoromethyl)phenoxy]-3-phenylpropylamine) is a selective serotonin (5-hydroxytryptamine) uptake inhibitor. Fluoxetine hydrochloride is marketed for the treatment of depression. This compound is among many taught in U.S. Patents Number 4,018,895, 4,194,009, and 4,314,081 as being potent, selective blockers of serotonin uptake.

Fluoxetine is a racemate of the two enantiomeric forms. The biological and pharmacological activity of each enantiomer has been found to be essentially the same; see, Robertson et al., J. Med. Chem., 31, 1412 (1988).

Norfluoxetine [3-(4-trifluoromethylphenoxy)-3-phenylpropylamine] is a metabolite of fluoxetine and is known to block monoamine uptake, especially serotonin. See U.S. Patent Number 4,313,896. Norfluoxetine has only been evaluated as the racemate, and since it is a metabolite of fluoxetine, it is believed that this compound contributes in part to the biological activity seen upon chronic administration of fluoxetine. Since the eudismic ratio for fluoxetine, i.e., the ratio of affinities or activities of its two enantiomers, is approximately unity, conventional wisdom would suggest that the individual enantiomers of norfluoxetine would similarly have equivalent activities. We have unexpectedly discovered that the (S)-enantiomer of norfluoxetine is substantially more active than its (R)-cognate.

Accordingly, this invention provides the compound (S)-norfluoxetine 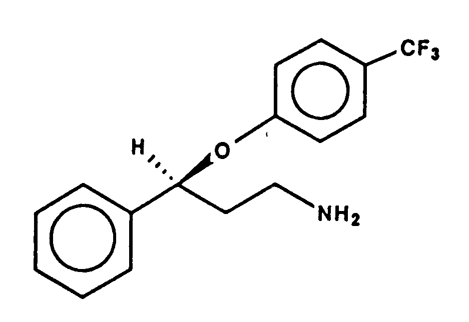
and pharmaceutically acceptable salts and solvates thereof.

The compounds of the invention can be used in a method for inhibiting serotonin uptake in mammals which comprises administering to a mammal requiring increased neurotransmission of serotonin an effective amount of (S)-norfluoxetine or a pharmaceutically acceptable salt or solvate thereof.

Further provided by this invention are pharmaceutical formulations comprising as an active ingredient (S)-norfluoxetine, or a pharmaceutically acceptable salt or solvate thereof, associated with one or more pharmaceutically acceptable carriers, diluents, or excipients therefor.

This invention includes the pharmaceutically acceptable salts of (S)-norfluoxetine. Since (S)-norfluoxetine is an amine, it is basic in nature and accordingly reacts with any number of inorganic and organic acids to form pharmaceutically acceptable acid addition salts. Acids commonly employed to form such salts include inorganic acids such as hydrochloric, hydrobromic, hydriodic, sulfuric and phosphoric acid, as well as organic acids such as para-toluenesulfonic, methanesulfonic, oxalic, para-bromophenylsulfonic, carbonic, succinic, citric, benzoic and acetic acid, and related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephathalate, sulfonate, xylenesulfonate, phenylacetate, phenylpropionate, phenylbutyrate, citrate, lactate, β-hydroxybutyrate, glycollate, maleate, tartrate, methanesulfonate, propanesulfonates, naphthalene-1-sulfonate, naphthalene-2-sulfonate, mandelate, hippurate, gluconate, lactobionate and the like salts. Preferred pharmaceutically acceptable salts include those formed with mineral acids such as hydrochloric acid and hydrobromic acid, and especially those formed with organic acids such as fumaric acid and maleic acid.

The pharmaceutically acceptable salts of (S)-norfluoxetine can also exist as various solvates, such as with water, methanol, ethanol, dimethylformamide, and the like. Mixtures of such solvates can also be prepared. The hydrates are particularly useful, especially those of the maleate salt of (S)-norfluoxetine. (S)-Norfluoxetine maleate hemihydrate is particularly useful because of its crystalline properties.

(S)-Norfluoxetine can be prepared by any of a number of methods generally known in the art. For example, there are several methods provided in the literature for making the racemate of norfluoxetine, see U.S. Patent 4,313,896. The racemate of norfluoxetine in turn can be resolved into its (S) and (R) components by standard methods. In particular, norfluoxetine can be reacted with an enantiomerically pure chiral derivatizing agent, resolved on the basis of the different physicochemical properties of the diastereomeric derivatives, and then converted to the two separate enantiomers of norfluoxetine. One particularly preferred method of accomplishing this derivatization is analogous to that described in Robertson et al., J. Med. Chem., 31, 1412 (1988) wherein fluoxetine was reacted with an optically active form of 1-(1-naphthyl)ethyl isocyanate to form a urea derivative of fluoxetine. A similar mixture of norfluoxetine derivatives can be separated through high pressure liquid chromatography into the individual diastereomers. Each individual diastereomer, in turn, can then be hydrolyzed to the individual enantiomers of norfluoxetine.

A preferred method of preparing (S)-norfluoxetine is similar to that labeled Scheme I in the Robertson et al. reference. (S)-(-)-3-Chloro-1-phenylpropanol (II) is either commercially available or can be prepared by chiral reduction of 3-chloropropiophenone II can be transformed into (S)-3-amino-1-phenylpropanol (III). Although a number of routes to convert the chloride intermediate to the amino compound are available, the preferred method is the transformation of the chloride to an intermediate N-substituted phthalimide which can be transformed to the desired primary amino intermediate III. This reaction sequence is a Gabriel synthesis wherein the potassium salt of phthalimide is reacted with (S)-(-)-3-chloro-1-phenylpropanol, preferably in the presence of a nonreactive solvent such as dimethylformamide or dimethylsulfoxide, to prepare the (S)-3-phthalimido-1-phenylpropanol intermediate. The phthalimido intermediate may be hydrolyzed to provide the desired amino intermediate III. However, to prevent the possible racemization of the intermediate, the phthalimide intermediate is preferably treated with hydrazine in a nonreactive solvent such as ethanol to provide the desired (S)-3-amino-1-phenylpropanol intermediate III. This latter compound can then be reacted with sodium hydride in dimethylsulfoxide or some other nonreactive solvent such as dimethylacetamide to generate the alkoxide which, upon treatment with 4-chloro- or 4-fluoro-benzotrifluoride, leads to a facile nucleophilic aromatic substitution to provide (S)-norfluoxetine.

Thus, in one embodiment of the present invention there is provided a process for preparing S-norfluoxetine, or a pharmaceutically-acceptable salt or solvate thereof, which comprises generating the anion of (S)-3-amino-1-phenyl-1-propanol (IV): 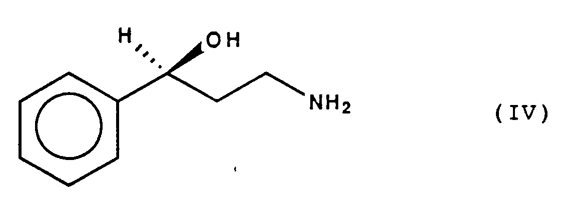
or an appropriate amino protected form thereof, so as to displace the leaving group X from a compound of formula (V): 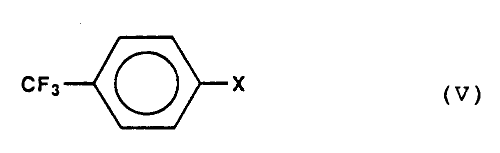
, removing any amino protecting groups which may be present, and salifying or solvating as appropriate.

The novel enantiomer of the invention may also be prepared by displacing the leaving group X from an (R)-isomer of formula (VI): 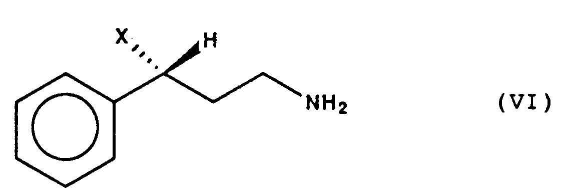
where X is a leaving group, or an amino protected form thereof, with the anion generated from the compound of formula (VII): 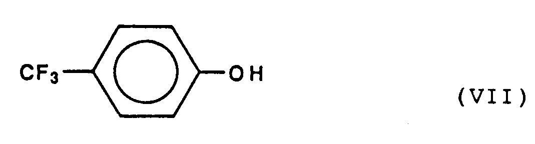
, removing any amino protecting groups which may be present in the product, and salifying or solvating as appropriate.

Typical amino protecting groups which may be mentioned are acyl or benzyl derivatives, which can be formed and removed in conventional fashion. The amino group can also be protected by forming an acid addition salt.

X is preferably halo, such as fluoro, chloro or bromo. The anion is preferably generated using sodium hydride. The displacement of the leaving group is preferably effected at temperatures from 50 to 100° C. Polar aprotic solvents are preferred, especially dimethylsulfoxide.

The (R)-isomer of formula (VI) can be prepared by reaction of the appropriate enantiomer of 3-amino-1-phenyl-1-propanol with halogenating agents such as SOCl₂, so as to prepare compounds where X is halo, with tosyl bromide or mesyl chloride so as to prepare a compound where Q is tosyloxy or mesyloxy. Any suitable inert organic solvent such as tetrahydrofuran methylene chloride could be used to effect the displacement. Suitable reaction temperature would range from 20 to 100° C.

The compound of the invention can also be prepared by aminating a compound of the formula (VIII): 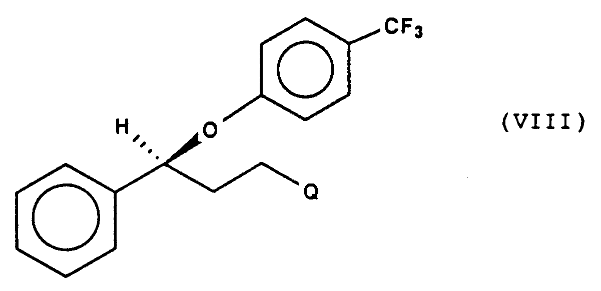
wherein Q is an appropriate leaving group, such as halo or tosyloxy.

The amination can be effected by reaction with ammonia dissolved in a suitable solvent such as ethanol or water, or by reaction with sodium azide followed by reduction using, for example, triphenylphosphine or nascent hydrogen. Suitable inert organic solvents such as ethanol can be used to effect the conversion. Suitable reaction temperatures for the reaction range from 20 to 100° C.

The compound of formula (VIII) can be prepared by generating the anion of a compound of formula (IX) 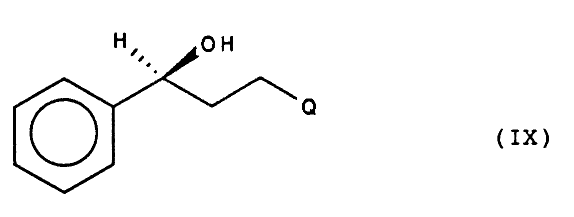
, for example with sodium hydride, and carrying out a nucleophilic displacement with the compound of formula (V).

The pharmaceutically acceptable acid addition salts of the invention are typically formed by reacting (S)-norfluoxetine with an equimolar or excess amount of acid. The reactants are generally combined in a mutual solvent such as diethyl ether or benzene, and the salt normally precipitates out of solution within about one minute to 10 days, and can be isolated by filtration.

(S)-Norfluoxetine according to the invention is substantially free from, i.e. is associated with less than 10%, preferably less than 7%, most preferably less than 5%, by weight, of the corresponding (R)-isomer.

The following non-limiting example further illustrates the invention.

### Example 1

### (S)-Norfluoxetine

### A. Preparation of (S)-3-phthalimido-1-phenylpropanol.

To a solution of 470 mg of (S)-(-)-3-chloro-1-phenylpropanol in 4 ml of dimethylformamide were added 612 mg of potassium phthalimide in 4 ml of dimethylformamide. The mixture was heated at 100°C for 6 hours, allowed to cool to room temperature, and stirred overnight. The mixture was filtered, the filtrate diluted with water, and the solution extracted with ethyl acetate. The organic layer was washed once with water, once with 0.2 N sodium hydroxide, once again with water, and once with a saturated solution of sodium chloride, dried over sodium sulfate, and concentrated in vacuo to provide an opaque oil that solidified. Crystallization from ethyl acetate/hexanes provided 350 mg of the title intermediate as a white powder, m.p. 80-82.5°C.

| Analysis for C₁₇H₁₅NO₃: | | | |
|---|---|---|---|
| Calc.: | C, 72.58; | H, 5.38; | N, 4.98; |
| Found: | C, 72.57; | H, 5.40; | N, 4.96. |

### B. Preparation of (S)-3-amino-1-phenyl-1-propanol.

To a solution of 4.046 g of (S)-3-phthalimido-1-phenyl-1-propanol in 100 ml of ethanol was added 2.5 ml of anhydrous hydrazine. The mixture was heated to reflux under a nitrogen atmosphere for 3.5 hours, cooled to room temperature, and allowed to stir overnight. The resulting precipitate was removed by filtration and the filtrate concentrated in vacuo. The resulting oil was treated with diethyl ether and 25 ml of 5 N sodium hydroxide. The layers were separated, and the organic layer was dried over sodium sulfate and concentrated in vacuo to provide 1.92 g of an opaque oil. Two hundred milligrams of this oil was treated with oxalic acid in ethyl acetate and crystallized from ethyl acetate/methanol to provide 210 mg of the title intermediate as the oxalate salt, m.p. 161-162°C.

| Analysis of the oxalate salt: C₁₁H₁₅NO₅: | | | |
|---|---|---|---|
| Calc.: | C, 54.77; | H, 6.27; | N, 5.81; |
| Found: | C, 54.96; | H, 6.15; | N, 5.79. |

### C. Preparation of (S)-norfluoxetine.

To a slurry of 484 mg of 60% sodium hydride in oil in 10 ml of dimethylacetamide were added 1.74 g of (S)-3-amino-1-phenyl-1-propanol in 40 ml of dimethylacetamide. The mixture was heated at 70°C for 10 minutes. 4-Fluorobenzotrifluoride (1.54 ml) was added to the reaction mixture and the solution heated at 100°C for 3 hours. The mixture was poured into ice water and extracted into diethyl ether. The organic extract was washed three times with water, once with a saturated sodium chloride solution, dried over sodium sulfate and concentrated in vacuo to provide 2.96 g of a yellow oil. The oil was purified by high pressure liquid chromatography over silica gel eluting with a gradient of methylene chloride to 10% methanol in methylene chloride to which 0.5% of ammonium hydroxide had been added. The desired fractions were combined and concentrated in vacuo to yield 1.5 g of the title product as an amber oil. The residue was dissolved in ethyl acetate and a solution of 451 mg of oxalic acid in ethyl acetate was added. The resulting precipitate was crystallized from ethyl acetate/methanol to provide 1.67 g of the desired title product as the oxalate salt, m.p. 148-150°C. In the same way were prepared (S)-norfluoxetine hydrochloride (m.p. 128-130°C), (S)-norfluoxetine fumarate (m.p. 156-157°C), and (S)-norfluoxetine maleate hemihydrate (m.p. 94-96°C) (in this case the reaction between the acid and the free base was effected in diethyl ethyl rather than ethyl acetate, and the product crystallized out from the reaction mixture on cooling).

| Analysis for C₁₆H₁₆F₃NO·HCl ((S)-norfluoxetine hydrochloride): | | | |
|---|---|---|---|
| Calc.: | C, 57.93; | H, 5.17; | N, 4.22; |
| Found: | C, 57.86; | H, 4.94; | N, 4.15. |

| Analysis for C₂₀H₂₀F₃NO₅ ((S)-norfluoxetine fumarate): | | | |
|---|---|---|---|
| Calc.: | C, 58.39; | H, 4.90; | N, 3.41; |
| Found: | C, 58.63; | H, 4.90; | N, 3.57. |

| Analysis for C₂₀H₂₀F₃NO₅ ((S)-norfluoxetine maleate hemihydrate, but block dried): | | | |
|---|---|---|---|
| Calc.: | C, 58.39; | H, 4.90; | N, 3.41; |
| Found: | C, 58.18; | H, 4.76; | N, 3.50. |

The above hemihydrate had been crystallized from diethyl ether and was found to contain 2.2% of water by thermogravimetric analysis prior to drying.

Crystallization of the (S)-norfluoxetine maleate hemihydrate from water provided crystalline (S)-norfluoxetine maleate hydrate (1:1), m.p. 97-101°C, calculated to have 3.6% water by thermogravimetric analysis.

Drying the hemihydrate at 45°C for one hour in a vacuum oven provided anhydrous (S)-norfluoxetine maleate, m.p. 96-97°C, thermogravimetric analysis indicating water in the amount of 0.02%.

According to the same procedure beginning with (R)-(+)-3-chloro-1-phenyl-1-propanol, (R)-norfluoxetine was prepared. The maleate salt of (R)-norfluoxetine had a melting point of 95-97°C.

(S)-Norfluoxetine and its pharmaceutically acceptable salts and solvates are useful for inhibiting the uptake of serotonin, and can be used in a method for inhibiting serotonin uptake in mammals which comprises administering to a mammal requiring increased neurotransmission of serotonin a pharmaceutically effective amount of (S)-norfluoxetine or a pharmaceutically acceptable salt thereof.

The term "pharmaceutically effective amount", as used herein, represents an amount of (S)-norfluoxetine which is capable of inhibiting serotonin uptake. The particular dose of compound administered according to this invention will, of course, be determined by the particular circumstances surrounding the case, including the route of administration, the particular condition being treated, and similar considerations. (S)-Norfluoxetine can be administered by a variety of routes including the oral, rectal, transdermal, subcutaneous, intravenous, intramuscular or intranasal routes. A typical daily dose will contain from about 0.01 mg/kg to about 20 mg/kg of (S)-norfluoxetine. Preferred daily doses will be about 0.05 to about 10 mg/kg, ideally about 0.1 to about 5 mg/kg.

(S)-Norfluoxetine has the ability to treat a variety of disorders in mammals associated with dysfunction in serotonergic systems such as obesity, bulemia, obsessive-compulsive disorders, depression, alcoholism, pain, premenstrual syndrome, loss of memory, anxiety, smoking, sleep apnea, and migraine. The compound can be used as an aid in increasing the rate of recanalization following thrombolytic or angioplasty therapy, and can be used to prevent restenosis or vasospasm following thrombolysis or angioplasty therapy. (S)-Norfluoxetine also has little effect on metabolism of concurrently administered drugs such as barbiturates or tricyclic antidepressants, unlike fluoxetine. (S)-Norfluoxetine is relatively non-toxic and has an excellent therapeutic index. Therefore, the present invention also provides methods of treating the above disorders at rates set forth above for inhibiting serotonin uptake in mammals.

The following experiment was conducted to demonstrate the ability of (S)-norfluoxetine to inhibit the uptake of serotonin as compared with its related enantiomer, racemate, and comparable fluoxetine analogs. This general procedure is set forth by Wong et al., in Drug Development Research 6:397-403 (1985).

Male Sprague-Dawley rats (110-150 g) from Harlan Industries (Cumberland, IN) were fed a Purina Chow ad libitum for at least 3 days before being used in the studies. Rats were killed by decapitation. Whole brains were removed and dissected. Cerebral cortex was homogenized in 9 volumes of a medium containing 0.32 M sucrose and 10 mM glucose. Crude synaptosomal preparations were isolated after differential centrifugation at 1,000 g for 10 min. and 17,000 g for 28 min. The final pellets were suspended in the same medium and kept in ice until use within the same day.

Synaptosomal uptake of ³H-serotonin (³H-5-hydroxytryptamine, ³H-5HT) was determined as follows. Cortical synaptosomes (equivalent to 1 mg of protein) were incubated at 37°C for 5 min in 1 ml of Krebs-. bicarbonate medium containing also 10 mM glucose, 0.1 mM iproniazid, 1 mM ascorbic acid, 0.17 mM EDTA, and 50 nM ³H-5HT. The reaction mixture was immediately diluted with 2 ml of ice-chilled Krebs-bicarbonate buffer and filtered under vacuum with a cell harvester (Brandel, Gaithersburg, MD). Filters were rinsed twice with approximately 5 ml of ice-chilled 0.9% saline and were transferred to a counting vial containing 10 ml of scintillation fluid (PCS, Amersham, Arlington Heights, IL). Radioactivity was measured by a liquid scintillation spectrophotometer. Accumulation of ³H-5HT at 4°C represented the background and was subtracted from all samples.

The results of the evaluation of (S)-norfluoxetine and related compounds from two side-by-side experiments are set forth below in Table I. In the Table, column 1 identifies the compound evaluated, and column 2 provides the concentration of the test compound at 10⁻⁹ M (nM) needed to inhibit 50% of serotonin (5HT) uptake and is indicated in the Table as the IC₅₀. The first experiment employed an older lot of tritiated 5HT whereas the second experiment employed a new lot of ³H-5HT.

**Table I**

| INHIBITION OF 5HT UPTAKE IN VITRO | | |
|---|---|---|
| Compound | 5HT IC₅₀(nM) | |
| | Expt. 1 | Expt. 2 |
| (R,S)-norfluoxetine | 202 | 55.8 |
| (R)-norfluoxetine | 1051 | 484.1 |
| (S)-norfluoxetine | 69 | 29.8 |
| (R,S)-fluoxetine | 79 | 34.4 |
| (R)-fluoxetine | 127 | 39.7 |
| (S)-fluoxetine | 93 | 25.0 |

The compound and salts and solvates of the present invention are preferably formulated prior to administration. Therefore, a preferred embodiment of the present invention is a pharmaceutical formulation comprising (S)-norfluoxetine, or a pharmaceutically acceptable salt or solvate thereof, associated with one or more pharmaceutically acceptable carriers, diluents or excipients therefor.

The present pharmaceutical formulations are prepared by known procedures using well known and readily available ingredients. In making the compositions of the present invention, the active ingredient will usually be mixed with a carrier, or diluted by a carrier, or enclosed within a carrier which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), ointments containing, for example, up to 10% by weight of the active compound, soft and hard gelatin capsules, suppositories, sterile injectable solutions and sterile packaged powders.

Some examples of suitable carriers, excipients, and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl-and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient by employing procedures well known in the art. Orally-acceptable formulations such as capsules or tablets are preferred.

The compositions are preferably formulated in a unit dosage form, each dosage containing from about 5 to about 500 mg, more usually about 25 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

The following non-limiting examples illustrate suitable formulations:

### Formulation 1

Hard gelatin capsules were prepared using the following ingredients:

| | Quantity (mg/capsule) |
|---|---|
| (S)-Norfluoxetine maleate hemihydrate | 1.44 |
| Starch powder | 226.6 |
| Silicone Fluid 350 CS | 2.0 |
| Total | 2̅3̅0̅.̅0̅4̅ mg |

The above ingredients were mixed and filled into hard gelatin capsules in 230.04 mg quantities. Each capsule contained the equivalent of 1 mg of (S)-norfluoxetine (base).

### Formulation 2

Twenty milligrams (equivalent (S)-Norfluoxetine base) containing capsules were prepared using the following ingredients:

| | Quantity (mg/capsule) |
|---|---|
| (S)-Norfluoxetine maleate hemihydrate | 28.78 |
| Starch powder | 199.2 |
| Silicone Fluid 350 CS | 2.00 |
| Total | 2̅2̅9̅.̅9̅8̅ mg |

The above ingredients were mixed and filled into hard gelatin capsules in 229.98 mg quantities. cooled to -30°C. and transferred to a filling device.

### Formulation 3

Tablets each containing 60 mg of active ingredient are made as follows:

| | |
|---|---|
| (S)-Norfluoxetine phosphate | 60 mg |
| starch | 45 mg |
| microcrystalline cellulose | 35 mg |
| polyvinylpyrrolidone (as 10% solution in water) | 4 mg |
| sodium carboxymethyl starch | 4.5 mg |
| magnesium stearate | 0.5 mg |
| talc | 1 mg |
| Total | 1̅5̅0̅ m̅g̅ |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

### Formulation 4

Suppositories each containing 225 mg of active ingredient may be made as follows:

| | |
|---|---|
| (S)-Norfluoxetine | 225 mg |
| saturated fatty acid glycerides | 2,000 mg |
| Total | 2̅,̅2̅2̅5̅ m̅g̅ |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

## Claims (Claims for the following Contracting State(s): DE, GB, FR, IT)

1. (S)-Norfluoxetine, or a pharmaceutically acceptable salt or solvate thereof.

2. (S)-Norfluoxetine, or a pharmaceutically acceptable salt thereof.

3. (S)-Norfluoxetine maleate hemihydrate.

4. A pharmaceutical formulation comprising as an active ingredient (S)-norfluoxetine, or a pharmaceutically acceptable salt or solvate thereof, associated with one or more pharmaceutically acceptable carriers, diluents or excipients therefor.

5. A pharmaceutical formulation as claimed in claim 4 comprising (S)-norfluoxetine or a pharmaceutically acceptable salt thereof as an active ingredient.

6. A pharmaceutical formulation as claimed in claim 4 wherein the active ingredient is (S)-norfluoxetine maleate hemihydrate.

7. A pharmaceutical formulation as claimed in any one of claims 4 to 6 in the form of a capsule or tablet.

8. (S)-Norfluoxetine, or a pharmaceutically-acceptable salt or solvate thereof, as claimed in any one of claim 1 to 3, or a pharmaceutical formulation as claimed in any one of claims 4 to 7, for use in inhibiting serotonin uptake in mammals.

9. (S)-Norfluoxetine, or a pharmaceutically acceptable salt or solvate thereof, as claimed in any one of claims 1 to 3, or a pharmaceutical formulation as claimed in any one of claims 4 to 7, for use in treating depression in humans.

10. (S)-Norfluoxetine, or a pharmaceutically acceptable salt or solvate thereof, as claimed in any one of claims 1 to 3, or a pharmaceutical formulation as claimed in any one of claims 4 to 7, for treating obesity in humans.

11. (S)-Norfluoxetine, or a pharmaceutically acceptable salt or solvate thereof, as claimed in any one of claims 1 to 3, or a pharmaceutical formulation as claimed in any one of claims 4 to 7, for suppressing the desire of humans to consume alcohol.

12. A process for preparing (S)-norfluoxetine, or a pharmaceutically-acceptable salt or solvate thereof, which comprises:
(A) generating the anion of (S)-3-amino-1-phenyl-1-propanol: 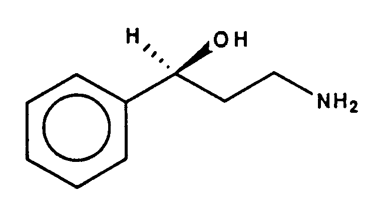 or an amino protected form thereof, so as to displace the leaving group X from a compound of formula: 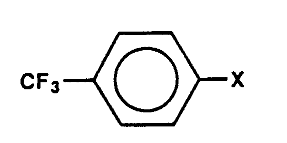 , removing any amino protecting groups which may be present in the product, and salifying or solvating as appropriate;
(B) displacing the leaving group X from an (R)-isomer of formula (VI): 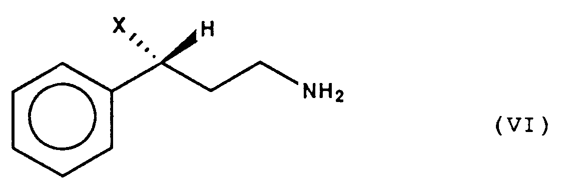 where X is a leaving group, or an amino protected form thereof, with the anion generated from the compound of formula (VII): 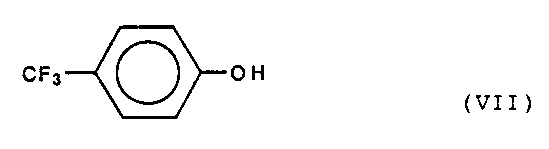 , removing any amino protecting groups which may be present in the product, and salifying or solvating as appropriate;
(C) aminating a compound of the formula (VIII): 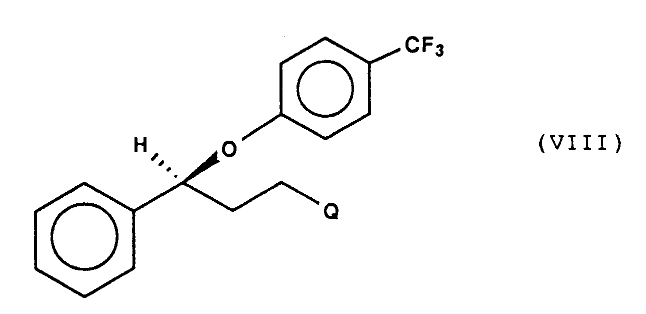 wherein Q is an appropriate leaving group; or
(D) resolving racemic norfluoxetine.

## Claims (Claims for the following Contracting State(s): ES)

1. A process for preparing (S)-norfluoxetine, or a pharmaceutically-acceptable salt or solvate thereof, which comprises:
(A) generating the anion of (S)-3-amino-1-phenyl-1-propanol: 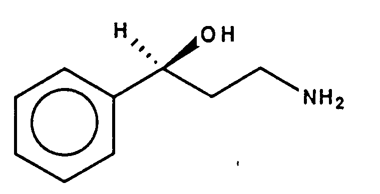 or an amino protected form thereof, so as to displace the leaving group X from a compound of formula: 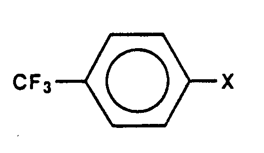 , removing any amino protecting groups which may be present in the product, and salifying or solvating as appropriate;
(B) displacing the leaving group X from an (R)-isomer of formula (VI): 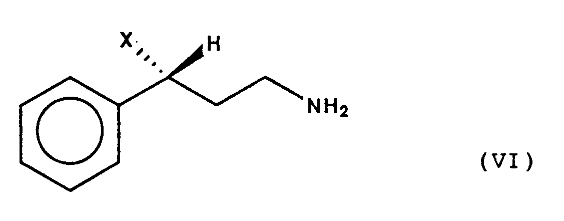 where X is a leaving group, or an amino protected form thereof, with the anion generated from the compound of formula (VII): 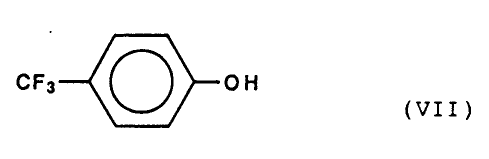 , removing any amino protecting groups which may be present in the product, and salifying or solvating as appropriate;
(C) aminating a compound of the formula (VIII): 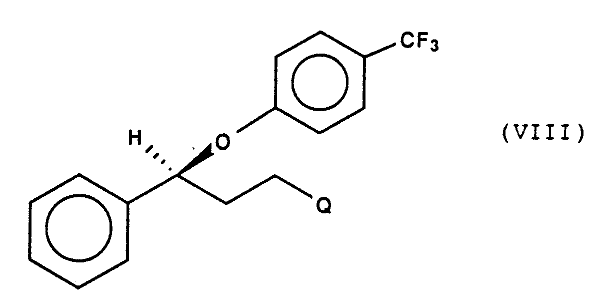 wherein Q is an appropriate leaving group; or
(D) resolving racemic norfluoxetine.

2. A process according to claim 1 for preparing (S)-norfluoxetine, or a pharmaceutically acceptable salt thereof.

3. A process according to claim 1 for preparing (S)-norfluoxetine maleate hemihydrate.

4. A process for preparing a pharmaceutical formulation comprising admixing (S)-norfluoxetine, or a pharmaceutically acceptable salt or solvate thereof, with one or more pharmaceutically acceptable carriers, diluents or excipients therefor.

## Patentansprüche (Patentansprüche für folgende(n) Vertragsstaat(en): DE, GB, FR, IT)

1. (S)-Norfluoxetin oder ein pharmazeutisch annehmbares Salz oder Solvat davon.

2. (S)-Norfluoxetin oder ein pharmazeutisch annehmbares Salz davon.

3. (S)-Norfluoxetinmaleathemihydrat.

4. Pharmazeutisches Präparat umfassend als aktiven Inhaltsstoff (S)-Norfluoxetin oder ein pharmazeutisch annehmbares Salz oder Solvat davon zusammen mit einem oder mehreren pharmazeutisch annehmbaren Trägern, Verdünnungsmitteln oder Hilfsstoffen dafür.

5. Pharmazeutisches Präparat nach Anspruch 4 umfassend (S)-Norfluoxetin oder ein pharmazeutisch annehmbares Salz davon als aktiven Inhaltsstoff.

6. Pharmazeutisches Präparat nach Anspruch 4, worin der aktive Inhaltsstoff (S)-Norfluoxetinmaleathemihydrat ist.

7. Pharmazeutisches Präparat nach einem der Ansprüche 4 bis 6 in Form einer Kapsel oder Tablette.

8. (S)-Norfluoxetin oder ein pharmazeutisch annehmbares Salz oder Solvat davon nach einem der Ansprüche 1 bis 3 oder ein pharmazeutisches Präparat nach einem der Ansprüche 4 bis 7 zur Verwendung zur Hemmung der Serotoninaufnahme bei Säugetieren.

9. (S)-Norfluoxetin oder ein pharmazeutisch annehmbares Salz oder Solvat davon nach einem der Ansprüche 1 bis 3 oder ein pharmazeutisches Präparat nach einem der Ansprüche 4 bis 7 zur Verwendung zur Behandlung von Depressionen bei Menschen.

10. (S)-Norfluoxetin oder ein pharmazeutisch annehmbares Salz oder Solvat davon nach einem der Ansprüche 1 bis 3 oder ein pharmazeutisches Präparat nach einem der Ansprüche 4 bis 7 zur Behandlung von Fettsucht bei Menschen.

11. (S)-Norfluoxetin oder ein pharmazeutisch annehmbares Salz oder Solvat davon nach einem der Ansprüche 1 bis 3 oder ein pharmazeutisches Präparat nach einem der Ansprüche 4 bis 7 zur Unterdrückung des Wunsches zum Konsumieren von Alkohol bei Menschen.

12. Verfahren zur Herstellung von (S)-Norfluoxetin oder eines pharmazeutisch annehmbaren Salzes oder Solvats davon umfassend, daß man
(A) das Anion von (S)-3-Amino-1-phenyl-1-propanol 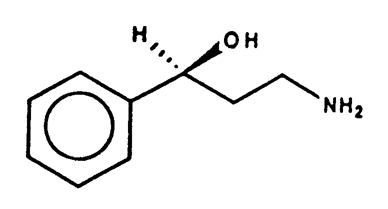 oder eine aminogeschützte Form davon erzeugt, um die Abgangsgruppe X einer Verbindung der Formel 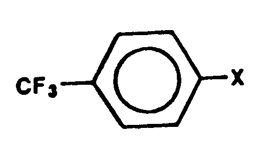 zu verdrängen, gegebenenfalls vorhandene Aminoschutzgruppen in dem Produkt entfernt und je nach Bedarf ein Salz bildet oder solvatisiert;
(B) die Abgangsgruppe X eines (R)-Isomers der Formel (VI): 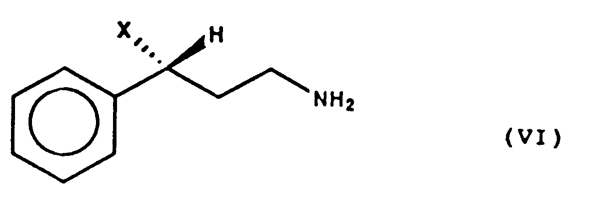 worin X eine Abgangsgruppe ist, oder einer aminogeschützten Form davon mit dem aus der Verbindung der Formel (VII): 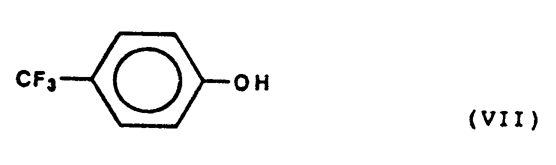 erzeugten Anion verdrängt, gegebenenfalls in dem Produkt vorhandene Aminoschutzgruppen entfernt und je nach Bedarf ein Salz bildet oder solvatisiert;
(C) eine Verbindung der Formel (VIII): 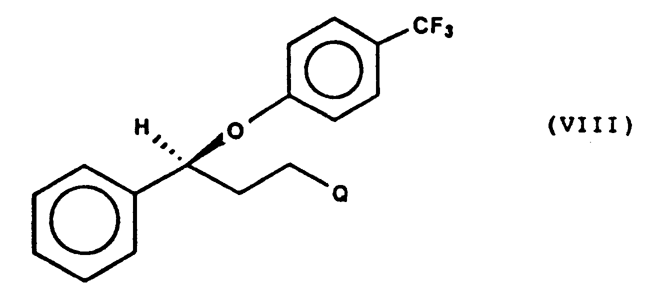 worin Q eine geeignete Abgangsgruppe ist, aminiert oder
(D) racemisches Norfluoxetin auftrennt.

## Patentansprüche (Patentansprüche für folgende(n) Vertragsstaat(en): ES)

1. Verfahren zur Herstellung von (S)-Norfluoxetin oder eines pharmazeutisch annehmbaren Salzes oder Solvats davon umfassend, daß man
(A) das Anion von (S)-3-Amino-1-phenyl-1-propanol 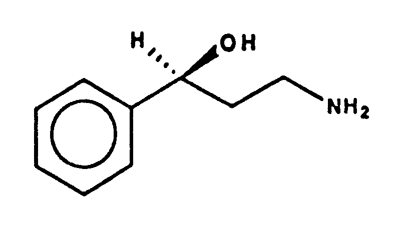 oder eine aminogeschützte Form davon erzeugt, um die Abgangsgruppe X einer Verbindung der Formel 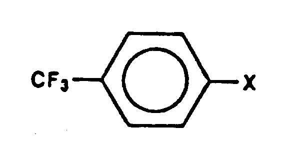 zu verdrängen, gegebenenfalls vorhandene Aminoschutzgruppen in dem Produkt entfernt und je nach Bedarf ein Salz bildet oder solvatisiert;
(B) die Abgangsgruppe X eines (R)-Isomers der Formel (VI): 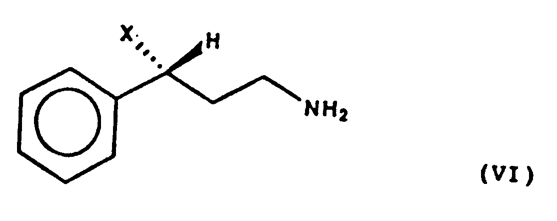 worin X eine Abgangsgruppe ist, oder einer aminogeschützten Form davon mit dem aus der Verbindung der Formel (VII): 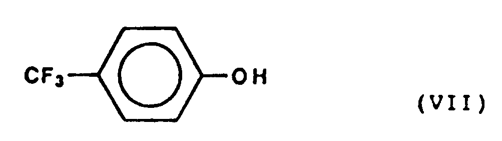 erzeugten Anion verdrängt, gegebenenfalls in dem Produkt vorhandene Aminoschutzgruppen entfernt und je nach Bedarf ein Salz bildet oder solvatisiert;
(C) eine Verbindung der Formel (VIII): 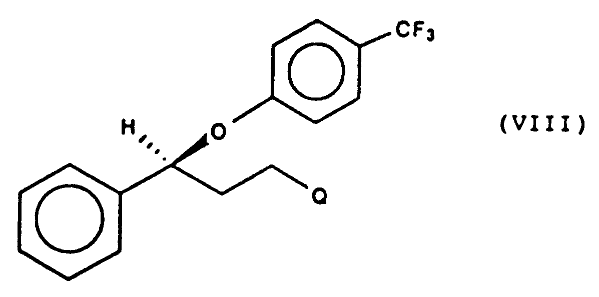 worin Q eine geeignete Abgangsgruppe ist, aminiert oder
(D) racemisches Norfluoxetin auftrennt.

2. Verfahren nach Anspruch 1 zur Herstellung von (S)-Norfluoxetin oder eines pharmazeutisch annehmbaren Salzes davon.

3. Verfahren nach Anspruch 1 zur Herstellung von (S)-Norfluoxetinmaleathemihydrat.

4. Verfahren zur Herstellung eines pharmazeutischen Präparates umfassend, daß man (S)-Norfluoxetin oder ein pharmazeutisch annehmbares Salz oder Solvat davon mit einem oder mehreren pharmazeutisch annehmbaren Trägern, Verdünnungsmitteln oder Hilfsstoffen dafür vermischt.

## Revendications (Revendications pour l'(les) Etat(s) contractant(s) suivant(s): DE, GB, FR, IT)

1. (S)-norfluoxétine ou un sel ou solvate pharmaceutiquement acceptable de celle-ci.

2. (S)-norfluoxétine, ou un sel pharmaceutiquement acceptable de celle-ci.

3. Hémihydrate du maléate de (S)-norfluoxétine.

4. Formulation pharmaceutique comprenant comme ingrédient actif la (S)-norfluoxétine, ou un sel ou un solvate pharmaceutiquement acceptable de celle-ci, associée à un ou à plusieurs supports, diluants ou excipients pharmaceutiquement acceptables pour celle-ci.

5. Formulation pharmaceutique selon la revendication 4, comprenant la (S)-norfluoxétine ou un sel pharmaceutiquement acceptable de celle-ci comme ingrédient actif.

6. Formulation pharmaceutique selon la revendication 4, dans laquelle l'ingrédient actif est l'hémihydrate du maléate de (S)-norfluoxétine.

7. Formulation pharmaceutique selon l'une quelconque des revendications 4 à 6 sous forme d'une capsule ou d'un comprimé.

8. (S)-norfluoxétine, ou un sel ou un solvate pharmaceutiquement acceptable de celle-ci, selon l'une quelconque des revendications 1 à 3, ou formulation pharmaceutique selon l'une quelconque des revendications 4 à 7, pour l'utilisation dans l'inhibition de l'absorption de la sérotonine chez les mammifères.

9. (S)-norfluoxétine, ou un sel ou un solvate pharmaceutiquement acceptable de celle-ci, selon l'une quelconque des revendications 1 à 3, ou formulation pharmaceutique selon l'une quelconque des revendications 4 à 7, pour l'utilisation dans le traitement de la dépression chez l'homme.

10. (S)-norfluoxétine, ou un sel ou un solvate pharmaceutiquement acceptable de celle-ci, selon l'une quelconque des revendications 1 à 3, ou formulation pharmaceutique selon l'une quelconque des revendications 4 à 7, pour le traitement de l'obésité chez l'homme.

11. (S)-norfluoxétine, ou un sel ou un solvate pharmaceutiquement acceptable de celle-ci, selon l'une quelconque des revendications 1 à 3, ou formulation pharmaceutique selon l'une quelconque des revendications 4 à 7, pour supprimer le désir chez l'homme de consommer de l'alcool.

12. Procédé pour la préparation de la (S)-norfluoxétine, ou d'un sel ou d'un solvate pharmaceutiquement acceptable de celle-ci, qui comprend :
(A) la génération de l'anion du (S)-3-amino-1-phényl-1-propanol : 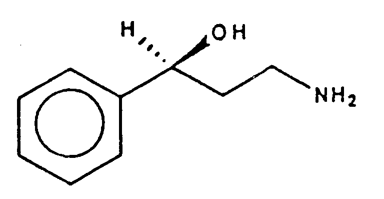 ou d'une forme amino-protégée de celui-ci, de façon à déplacer le groupe sortant X d'un composé répondant à la formule : 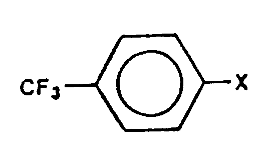 élimination de tous les groupes amino-protecteurs qui peuvent être présents dans le produit et la salification ou la solvatation d'une manière appropriée;
(B) le déplacement du groupe sortant X à partir d'un isomère (R) répondant à la formule (VI) 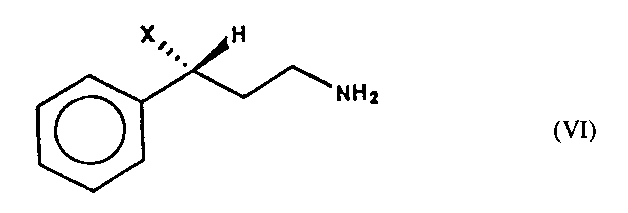 où X est un groupe sortant, ou une forme amino-protégée de celui-ci, avec l'anion généré à partir du composé répondant à la formule (VII) : 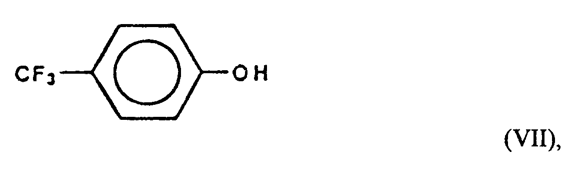 élimination de tous les groupes amino-protecteurs qui peuvent être présents dans le produit et la salification ou la solvatation d'une manière appropriée;
(C) l'amination d'un composé répondant à la formule (VIII): 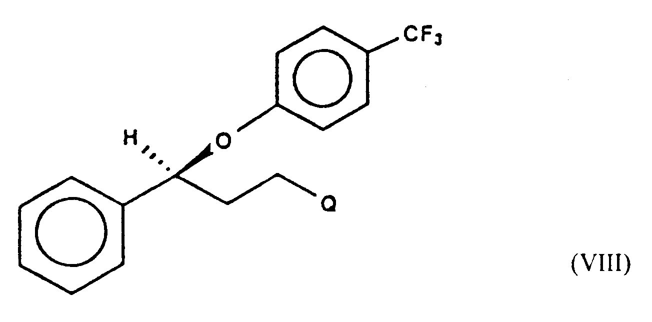 dans laquelle Q est un groupe sortant approprié; ou
(D) la résolution de la norfluoxétine racémique.

## Revendications (Revendications pour l'(les) Etat(s) contractant(s) suivant(s): ES)

1. Procédé pour la préparation de la (S)-norfluoxétine, ou d'un sel ou d'un solvate pharmaceutiquement acceptable de celle-ci, qui comprend :
(A) la génération de l'anion du (S)-3-amino-1-phényl-1-propanol : 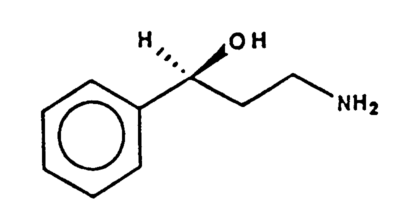 ou d'une forme amino-protégée de celui-ci, de façon à déplacer le groupe sortant X d'un composé répondant à la formule : 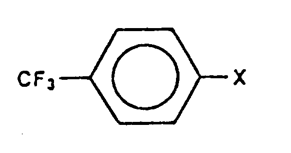 élimination de tous les groupes amino-protecteurs qui peuvent être présents dans le produit et la salification ou la solvatation d'une manière appropriée;
(B) le déplacement du groupe sortant X à partir d'un isomère (R) répondant à la formule (VI) 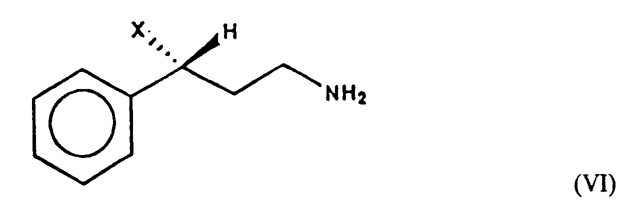 où X est un groupe sortant, ou une forme amino-protégée de celui-ci, avec l'anion généré à partir du composé répondant à la formule (VII) : 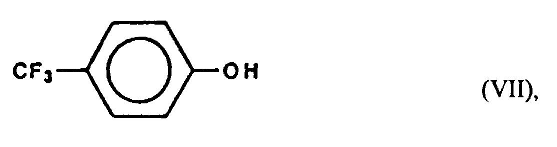 élimination de tous les groupes amino-protecteurs qui peuvent être présents dans le produit et la salification ou la solvatation d'une manière appropriée;
(C) l'amination d'un composé répondant à la formule (VIII): 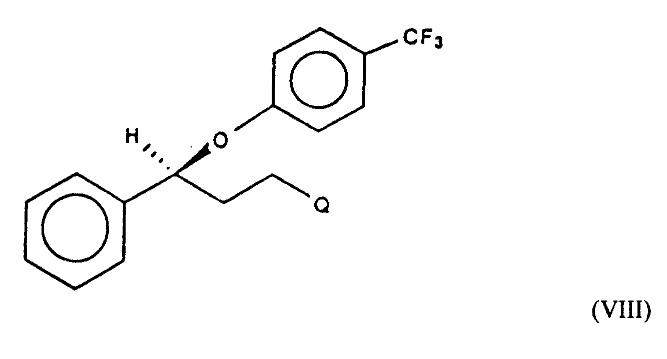 dans laquelle Q est un groupe sortant approprié; ou
(D) la résolution de la nofluoxétine racémique.

2. Procédé selon la revendication 1 pour la préparation de la (S)-norfluoxétine, ou d'un sel pharmaceutiquement acceptable de celle-ci.

3. Procédé selon la revendication 1 pour la préparation de l'hémihydrate du maléate de la (S)-norfluoxétine.

4. Procédé pour la préparation d'une formulation pharmaceutique comprenant le mélange de la (S)-norfluoxétine, ou d'un sel ou d'un solvate pharmaceutiquement acceptable de celle-ci, avec un ou plusieurs supports, diluants ou excipients pharmaceutiquement acceptables pour celle-ci.
